(19) Europäisches Patentamt
European Patent Office
Office européen des brevets

(11) Veröffentlichungsnummer: **0 345 615**
**A2**

(12) # EUROPÄISCHE PATENTANMELDUNG

(21) Anmeldenummer: **89109771.9**

(22) Anmeldetag: **30.05.89**

(51) Int. Cl.⁴: **C12N 15/00**

Patentansprüche für folgenden Vertragsstaat: ES.

(30) Priorität: **08.06.88 DE 3819463**

(43) Veröffentlichungstag der Anmeldung:
**13.12.89 Patentblatt 89/50**

(84) Benannte Vertragsstaaten:
**AT BE CH DE ES FR GB GR IT LI LU NL SE**

(71) Anmelder: **BEHRINGWERKE Aktiengesellschaft**
**Postfach 1140**
**D-3550 Marburg 1(DE)**

(72) Erfinder: **Amann, Egon, Dr.**
**Sachsenring 8**
**D-3550 Marburg(DE)**
Erfinder: **Abel, Karl-Josef, Dr.**
**Am Ziegenberg 6**
**D-3550 Marburg(DE)**

(74) Vertreter: **Meyer-Dulheuer, Karl-Hermann, Dr. et al**
**HOECHST Aktiengesellschaft Postfach 3540**
**D-6200 Wiesbaden(DE)**

(54) **Expressionsvektoren zur Herstellung unfusionierter Proteine in Mikroorganismen.**

(57) Expressionsvektoren werden beschrieben, die den trc-Promoter, die lacZ Ribosomenbindungsstelle eine NcoI-Schnittstelle mit einem Translationsinitiationscodon, einen Restriktionsenzymschnittstellen-Polylinker in allen drei Leserastern sowie Transkriptionsterminatoren enthalten. Die vorstehend genannten Elemente sind dabei in ein Plasmidrückgrat mit Replikationsorigin, Antibiotikaresistenzgen und dem lacI$^q$ Allel integriert. Solche Vektoren sind in besonderem Maße geeignet, für E.coli toxische Genprodukte zu exprimieren.

EP 0 345 615 A2

**Expressionsvektoren zur Herstellung unfusionierter Proteine in Mikroorganismen.**

Die Signale für die Initiation der Transkription und der Translation sind in prokaryotischen und eukaryotischen Genen verschieden. Aus diesem Grunde beobachtet man in der Regel keine oder nur eine geringe Expression, wenn man eukaryotische Gene in prokaryotische Zellen transferiert. Um eine effiziente Expression eukaryotischer Gene in Prokaryoten zu erreichen, werden diese häufig an 5′ Anteile prokaryotischer Gene ligiert, welches - vorausgesetzt, die beiden Translationsleserahmen stimmen überein - in der Synthese von Fusionsproteinen resultiert. Durch BrCN-Spaltung, Säurespaltung oder auch durch Einfügen von spezifischen Protease-Schnittstellen zwischen den beiden Protein-Anteilen des Fusionsproteins und anschließender Proteasebehandlung läßt sich dann aus dem Fusionsprotein der gewünschte eukaryontische Proteinanteil herauslösen. Dies ist in der Regel nur für kleinere Proteine möglich, da bei größeren Proteinen BrCN-Spaltung und Säurespaltung aufgrund der hohen Zahl der resultierenden Proteinfragmente nicht in Frage kommen. Die Proteasespaltung hat häufig das Problem der geringen Ausbeuten des gewünschten Proteins bzw. der hohen Kosten für die Protease, die zudem nur sehr rein eingesetzt werden kann. Die vorliegende Erfindung hat sich deshalb als Aufgabe gestellt, Fremdproteine in Prokaryonten mittels besonders geeigneter Expressionsvektoren in unfusioniertem Zustand durch direkte Expression in hoher Ausbeute zu erhalten.

Die Expression eines klonierten Genes erfordert seine effiziente Transkription und eine ebensolche Translation seiner mRNA. Letzteres benötigt die Anwesenheit einer bestimmtem Sequenz in der mRNA, welche als "Ribosomenbindungsstelle" (RBS) bezeichnet wird. Diese Stelle besteht aus einer 3-9 Basenpaar langen Region, die als "Shine- Dalgarno-Sequenz (SD) bezeichnet wird (Shine und Dalgarno (1975) Nature 254, 34-38) und welcher in einem Abstand von 3-11 Basenpaare ein ATG-Initiationscodon folgt, welches das aminoterminale Methionin kodiert. Die SD Sequenz ist komplementär zum 3′ Ende der 16S rRNA und wahrscheinlich kommt es zur Duplexbildung dieser beiden RNA Sequenzen, was wiederum als Voraussetzung für die Initiation der Translation angesehen wird (Steitz (1979) in: Biological Regulation and Control, R. Golderger ed., New York, Plenum Press). Zur Expression eines heterologen unfusionierten Proteins müssen deshalb in der Regel komplizierte Manipulationen unmittelbar stromaufwärts des eukaryotischen ATG-Initiationscodons vorgenommen werden. So muß z. B. die fehlende prokaryotische RBS vorgeschaltet werden, um eine effiziente Bindung des 5′ Endes der mRNA mit dem 3′ Ende der 16S ribosomalen RNA zu gewährleisten. Die Kombination der fusionierten prokaryotischen SD Sequenz mit dem eukaryotischen ATG Startcodon und einigen Basen der nachfolgenden Kodierungssequenz wird als "hybrid ribosome binding site" bezeichnet. Sowohl der Abstand der prokaryotischen SD-Sequenz zum ATG-Initiationscodon als auch die Basenzusammensetzung stromaufwärts und stromabwärts der SD Sequenz stellen kritische Parameter bei der Maximierung der Expression dar. Da es bisher keine verläßlichen Computerprogramme zur Vorhersage der idealen Sequenz der "hybrid ribosome binding site" für eine Hochexpression gibt, muß diese experimentell ermittelt werden. Dies kann zum einen durch Anwendung genetischer Methoden mit Hilfe der ß-Galaktosidase geschehen (Guarente et al. (1980) Cell 20, 543-533), zum anderen durch den Einsatz synthetischer Oligodesoxynukleotide. Letzteres erfordert häufig die empirische Testung vieler verschiedener Sequenzen, um eine maximale Expression des gewünschten Genproduktes zu erreichen.

Eines der am höchsten exprimierten Gene in E. coli ist das Produkt des lacZ Gens, die ß-Galaktosidase. In haploiden Stämmen kann die Menge an ß-Galaktosidase bis zu 5% des totalen Proteins ausmachen, in partiell diploiden Stämmen bis zu 20% (Zabin und Fowler (1978) in: The Operon, Miller, Reznikoff, ed., Cold Spring Harbor Laboratory). Die vorliegende Erfindung geht nun von der Annahme aus, daß ein hochexprimiertes Protein wie die ß-Galaktosidase auch eine für die Hochexpression optimierte mRNA hat und dort im besonderen eine optimierte RBS besitzt. Die Erfindung nutzt die auf "natürliche" Weise auf eine hohe Expression hin selektierte RBS der lacz mRNA in Verbindung mit einer stromabwärts eingefügten synthetischen DNA Sequenz, um eine hohe Ausbeute an exprimiertem Fremdprotein in unfusionierter Form zu erreichen. Die folgende Darstellung vergleicht die relevante 5′ Region der natürlichen lacZ Sequenz (Gilbert und Maxam (1973) Proc. Natl. Acad. Sci. USA 70, 3581-3584; Maizels (1973) Proc. Natl Acad. Sci. USA 70, 3585-3589; Dickson et al. (1975) Science 187, 27-35) mit der entsprechenden Sequenz der neuen Vektoren:

Sequenz der lacZ DNA: 5' CAATTTCACACAGGAAACAGCT ATG A 3'

||||||||||||||||||||·········

Sequenz der Vektor DNA: 5' CAATTTCACACAGGAAACAGACC ATG G 3'

Die synthetische DNA, die als Oligodesoxynucleotid-Doppelstrang hinter die lacZ RBS eingefügt wurde, hat die Sequenz:

5′ CCATGG 3′

3′ GGTACC 5′

und somit die Erkennungsregion für die Restriktionsendonuklease NcoI. Die synthetische DNA ist so positioniert, daß das 'ATG'-Initiationscodon, welches in der NcoI-Erkennungsstelle enthalten ist, in einem für die Hochexpression günstigem Abstand zur prokaryotischen RBS liegt. Die NcoI Stelle kommt im Plasmid nur einmal vor.

Die NcoI Stelle kann nun zur Hochexpression von Fremdgenen auf verschiedene Weise genutzt werden. Ein Aspekt der Erfindung besteht darin, daß die Plasmidvektoren zur Direktexpression eukaryotischer Gene besonders geeignet sind. Dies beruht darauf, daß zahlreiche eukaryotische Gene ebenfalls eine NcoI Stelle an ihrem ATG-Initiationscodon aufweisen. Die Consensus-Sequenz der 5′ Region unmittelbar vor dem ATG-Initiationscodon eukaryotischer mRNA wird von Kozak (Marilyn Kozak (1987) Nucleic Acids Research 15, 8125-8148) mit:

G

(GCC)GCCACCATGG

---

angegeben. Von 699 von Kozak erfaßten Sequenzen eukaryotischer mRNA tragen 124 (=17,7%) eine NcoI Stelle an ihrem ATG-Initiationscodon. Diese können direkt in den neuen Vektoren exprimiert werden. In 194 von den verbleibenden 575 Fällen (=33,7%) beginnt das zweite Codon mit einem "G" (mögliche Aminosäuren an dieser Position: Gly, Glu, Asp, Ala, Val). Die DNA Sequenzen dieser 33,7% können unter Beibehaltung der Aminosäuresequenz durch Mutagenese vor dem ATG in eine NcoI Stelle umgewandelt werden. Somit kann in 51,4% aller von Kozak untersuchten 699 eukaryotischen Gene entweder direkt (17,7%) oder nach Mutagenese (33,7%) DNA Fragmente erhalten werden, die in den neuen prokarytischen Vektoren kloniert und zur Expression gebracht werden können. Die Expressionsprodukte sind unfusioniert und in ihrer Aminosäuresequenz unverändert. Die verbleibenden 48,6% der eukaryotischen Gene können natürlich auch nach Einführung einer NcoI Stelle durch Mutagenese in den neuen Vektoren exprimiert werden, jedoch tritt in diesen Fällen eine Veränderung der zweiten Aminosäure von x nach Gly, Glu, Asp oder Ala auf.

Eine weitere Möglichkeit der Klonierung in den neuen Vektoren ergibt sich durch Schneiden der Sequenz mit NcoI und nachfolgendem enzymatischem Auffüllen des überlappenden Endes mittels Klenow-Polymerase. Man erhält so ein exponiertes 'ATG':

5′ CCATG 3′

3′ GGTAC 5′

An dieses exponierte 'ATG' kann nun jede Fremd-DNA, die ebenfalls ein glattes Ende hat, ligiert werden, wobei kein eigenes ATG mehr vorhanden sein muß. Trägt diese DNA die vollständige Information oder Teile der Information eines Strukturgenes, so können diese, sofern sie im korrekten Leserahmen zum manipulierten 'ATG' Vektorcodon stehen, exprimiert werden. Die spezielle Natur dieser Klonierung erlaubt, wie bereits oben beschrieben, die Expression des gewünschten Proteins ohne zusätzliche oder veränderte Aminosäuren. Eine weitere Anwendungsmöglichkeit ergibt sich aus der Verwendung handelsüblicher NcoI Linker, die in verschiedenen Längen (8-mer, 10mer, 12mer) erhältlich sind (Analects Vol. 14, No. 1; Fa. Pharmacia) und genutzt werden können, um den "offenen Leserahmen" eines beliebigen Strukturgens mit dem ATG Initiationscodon der neuen Vektoren zu kombinieren. Die Anwendung von NcoI Linkern erlaubt auch den Einsatz der neuen Vektoren zur Expression von cDNA Banken zum Immunscreenen mit bekannten Methoden (z. B. Broome und Gilbert (1978) Proc. Natl. Acad. Sci. USA 75, 2746-2749; Erlich et al. (1978) Cell 13, 681-689; Kemp und Cowman (1981) Proc. Natl. Acad. Sci. USA 78, 4520-4524).

Um eine Vielzahl von Restriktionsschnittstellen stromabwärts der NcoI Stelle zur Verfügung zu stellen, wurde die EcoRI-HindIII Polylinkerregion des handelsüblichen Vektors pUC18 hinter der NcoI Stelle in die Plasmidvektoren eingeführt (siehe Fig.). Als Folge können die verschiedensten Schnittstellen in der 3′ Region des zu exprimierenden Gens genutzt werden, um eine gerichtete Klonierung zu garantieren.

In einigen speziellen Beispielen der Anwendungen der neuen Vektoren kann auch die EcoRI Stelle benutzt werden, um ein Fremdprotein unfusioniert zu exprimieren. Dazu kann die Vektor DNA mit EcoRI geschnitten werden und das überhängende Einzelstrangende mit einzelstrang-spezifischen Nukleasen, z.B. mit der Mung Bean Nuklease, abverdaut werden. Dies resultiert in folgenden Sequenzen:

```
5' AACAGACCATGG  3'        in  pTrc99A
       ---

5' AACAGACCATGGG  3'       in  pTrc99B
       ---

5' AACAGACCATGGGG  3'      in  pTrc99C
       ---
```

An diese Enden kann nun eine Fremd-DNA mit glatten Enden so ligiert werden, daß der Leserahmen mit dem durch die Vektor DNA vorgegebenen ATG Startcodon übereinstimmt. Diese Manipulation kann im Einzelfall dazu führen, daß keine fremden Aminosäuren am N-Terminus des exprimierten heterologen Proteins vorkommen. In anderen Einzelfällen kann hinter dem Initiatormethionin eine fremde Aminosäure N-terminal an das heterologe Protein fusioniert vorkommen.

Eine weitere Anwendungsmöglichkeit der neuen Vektoren ergibt sich aus der Tatsache, daß der oben genannte Polylinker in allen drei Leserahmen in Bezug auf das ATG-Initiationscodon der NcoI Stelle zur Verfügung steht. Daraus folgt, daß DNA Fragmente von Strukturgenen, die durch Hydrolyse von in der Polylinkerregion vorkommenden Restriktionsenzymen erhalten wurden, im korrekten Leserahmen zum Vektor-ATG kloniert und exprimiert werden können. Diese Anwendungsmöglichkeit gilt jedoch nur für den Fall, daß die Anwesenheit einiger weniger fremder vektorkodierter Aminosäuren am N-Terminus des gewünschten Proteins nicht stört. Für diese Art der "kurzen Fusionsexpression" bieten die neuen Vektoren zumindest den Vorteil der ca. 5-10 fach erhöhten Expressionsrate im Vergleich zu ähnlichen lac-Promoter Vektoren (De Boer et al. (1983) Proc. Natl. Acad. Sci. USA 80, 21-25; Amann et al. (1983) Gene 25, 167-178).

Kurze Beschreibung der Vektoren

Die Vektoren besitzen alle den trc Promoter, ein Derivat des tac Promoters mit vergleichbar hoher Aktivität (Mulligan et al. (1985) J. Biol. Chem. 260, 3529-3538; Brosius et al. (1985) J. Biol. Chem. 260, 3539-3541). Tac und trc Promoter unterscheiden sich in einer einzigen Base: Während der tac Promoter einen Abstand zwischen -35 und -10 Region von 16 Basenpaaren aufweist, sind diese beiden Regionen im trc Promoter 17 Basenpaare voneinander entfernt. Der tac Promoter und seine Aktivität im Vergleich zu lac, trp und tet Promotoren wurde bereits beschrieben (De Boer et al. (1983) Proc. Natl. Acad. Sci. USA 80, 21-25; Russel und Bennett (1982) Gene 20, 231-243; Amann et al. (1983) Gene 25, 167-178). Die Vektoren benutzen stromabwärts des trc Promoters die lacZ Ribosomenbindungsstelle, gefolgt von einer NcoI Stelle, die ein Translationsinitiationscodon beinhaltet. Weiterhin folgt der EcoRI-HindIII Polylinker aus pUC18 sowie die Transcriptionsterminatoren des rrnB Operons (Brosius et al. (1981) J. Mol. Biol. 148, 107-127). Das "Plasmidrückgrad" besteht aus einem pBR322 Anteil, der einen ColE1-Typ Replikationsorigin sowie das Ampicillin-Resistenzgen (bla Gen) umfaßt. Da in den Vektoren die pBR322 DNA bis zur Position 2066 (PvuII Stelle) und damit Teile der rop Region deletiert wurde, kommt es zu einer Erhöhung der Plasmid-Kopie-Zahl (Balban et al. (1986) Gene 50, 3-40; Twigg und Sheratt (1980) Nature 283, 216-218), was sich positiv auf die Expressionshöhe auswirkt ("Gendosiseffekt"). In einigen $lacI^q$ Wirtsstämmen beobachtet man partielle Derepression des trc Promoters, was auf die erhöhte Kopiezahl der neuen Vektoren im Vergleich zu den bekannten tac Promoter Vektoren ptac11 und ptac12 (Amann et al. (1983) Gene 25, 167-168) zurückzuführen ist. Aus diesem Grunde wurden die erfindungsgemäßen Vektoren der pTrc99er Serie konstruiert, die als zusätzliche Information das $lacI^q$ Allel tragen. Diese Vektoren zeigen auch in E.coli Wirtsstämmen, die kein lacI$^q$ Allel tragen, vollständige Repression des trc Promoters in Abwesenheit von Induktor. Aus diesem Grunde sind diese Vektoren in besonderem Maße geeignet, für E.coli toxische Genprodukte zu exprimieren, da während der Klonierung und Anzuchtphase keine basale Expression mit der Folge der negativen Selektion auftritt. Die Tabelle zeigt die vollständige Nukleotidsequenz für pTrc99A:

Tabelle - 8 -

| | | | | | |
|---|---|---|---|---|---|
| 1 | GTTTGACAGC | TTATCATCGA | CTGCACGGTG | CACCAATGCT | TCTGGCGTCA |
| 51 | GGCAGCCATC | GGAAGCTGTG | GTATGGCTGT | GCAGGTCGTA | AATCACTGCA |
| 101 | TAATTCGTGT | CGCTCAAGGC | GCACTCCCGT | TCTGGATAAT | GTTTTTTGCG |
| 151 | CCGACATCAT | AACGGTTCTG | GCAAATATTC | TGAAATGAGC | TGTTGACAAT |
| 201 | TAATCATCCG | GCTCGTATAA | TGTGTGGAAT | TGTGAGCGGA | TAACAATTTC |
| 251 | ACACAGGAAA | CAGACCATGG | AATTCGAGCT | CGGTACCCGG | GGATCCTCTA |
| 301 | GAGTCGACCT | GCAGGCATGC | AAGCTTGGCT | GTTTTGGCGG | ATGAGAGAAG |
| 351 | ATTTTCAGCC | TGATACAGAT | TAAATCAGAA | CGCAGAAGCG | GTCTGATAAA |
| 401 | ACAGAATTTG | CCTGGCGGCA | GTAGCGCGGT | GGTCCCACCT | GACCCCATGC |
| 451 | CGAACTCAGA | AGTGAAACGC | CGTAGCGCCG | ATGGTAGTGT | GGGGTCTCCC |
| 501 | CATGCGAGAG | TAGGGAACTG | CCAGGCATCA | AATAAAACGA | AAGGCTCAGT |
| 551 | CGAAAGACTG | GGCCTTTCGT | TTTATCTGTT | GTTTGTCGGT | GAACGCTCTC |
| 601 | CTGAGTAGGA | CAAATCCGCC | GGGAGCGGAT | TTGAACGTTG | CGAAGCAACG |
| 651 | GCCCGGAGGG | TGGCGGGCAG | GACGCCCGCC | ATAAACTGCC | AGGCATCAAA |
| 701 | TTAAGCAGAA | GGCCATCCTG | ACGGATGGCC | TTTTTGCGTT | TCTACAAACT |
| 751 | CTTTTTGTTT | ATTTTTCTAA | ATACATTCAA | ATATGTATCC | GCTCATGAGA |
| 801 | CAATAACCCT | GATAAATGCT | TCAATAATAT | TGAAAAAGGA | AGAGTATGAG |
| 851 | TATTCAACAT | TTCCGTGTCG | CCCTTATTCC | CTTTTTTGCG | GCATTTTGCC |
| 901 | TTCCTGTTTT | TGCTCACCCA | GAAACGCTGG | TGAAAGTAAA | AGATGCTGAA |
| 951 | GATCAGTTGG | GTGCACGAGT | GGGTTACATC | GAACTGGATC | TCAACAGCGG |
| 1001 | TAAGATCCTT | GAGAGTTTTC | GCCCCGAAGA | ACGTTTTCCA | ATGATGAGCA |
| 1051 | CTTTTAAAGT | TCTGCTATGT | GGCGCGGTAT | TATCCCGTGT | TGACGCCGGG |
| 1101 | CAAGAGCAAC | TCGGTCGCCG | CATACACTAT | TCTCAGAATG | ACTTGGTTGA |
| 1151 | GTACTCACCA | GTCACAGAAA | AGCATCTTAC | GGATGGCATG | ACAGTAAGAG |
| 1201 | AATTATGCAG | TGCTGCCATA | ACCATGAGTG | ATAACACTGC | GGCCAACTTA |
| 1251 | CTTCTGACAA | CGATCGGAGG | ACCGAAGGAG | CTAACCGCTT | TTTTGCACAA |
| 1301 | CATGGGGGAT | CATGTAACTC | GCCTTGATCG | TTGGGAACCG | GAGCTGAATG |
| 1351 | AAGCCATACC | AAACGACGAG | CGTGACACCA | CGATGCCTAC | AGCAATGGCA |

1401 ACAACGTTGC GCAAACTATT AACTGGCGAA CTACTTACTC TAGCTTCCCG
1451 GCAACAATTA ATAGACTGGA TGGAGGCGGA TAAAGTTGCA GGACCACTTC
1501 TGCGCTCGGC CCTTCCGGCT GGCTGGTTTA TTGCTGATAA ATCTGGAGCC
1551 GGTGAGCGTG GGTCTCGCGG TATCATTGCA GCACTGGGGC CAGATGGTAA
1601 GCCCTCCCGT ATCGTAGTTA TCTACACGAC GGGGAGTCAG GCAACTATGG
1651 ATGAACGAAA TAGACAGATC GCTGAGATAG GTGCCTCACT GATTAAGCAT
1701 TGGTAACTGT CAGACCAAGT TTACTCATAT ATACTTTAGA TTGATTTAAA
1751 ACTTCATTTT TAATTTAAAA GGATCTAGGT GAAGATCCTT TTTGATAATC
1801 TCATGACCAA AATCCCTTAA CGTGAGTTTT CGTTCCACTG AGCGTCAGAC
1851 CCCGTAGAAA AGATCAAAGG ATCTTCTTGA GATCCTTTTT TTCTGCGCGT
1901 AATCTGCTGC TTGCAAACAA AAAAACCACC GCTACCAGCG GTGGTTTGTT
1951 TGCCGGATCA AGAGCTACCA ACTCTTTTTC CGAAGGTAAC TGGCTTCAGC
2001 AGAGCGCAGA TACCAAATAC TGTCCTTCTA GTGTAGCCGT AGTTAGGCCA
2051 CCACTTCAAG AACTCTGTAG CACCGCCTAC ATACCTCGCT CTGCTAATCC
2101 TGTTACCAGT GGCTGCTGCC AGTGGCGATA AGTCGTGTCT TACCGGGTTG
2151 GACTCAAGAC GATAGTTACC GGATAAGGCG CAGCGGTCGG GCTGAACGGG
2201 GGGTTCGTGC ACACAGCCCA GCTTGGAGCG AACGACCTAC ACCGAACTGA
2251 GATACCTACA GCGTGAGCTA TGAGAAAGCG CCACGCTTCC CGAAGGGAGA
2301 AAGGCGGACA GGTATCCGGT AAGCGGCAGG GTCGGAACAG GAGAGCGCAC
2351 GAGGGAGCTT CCAGGGGGAA ACGCCTGGTA TCTTTATAGT CCTGTCGGGT
2401 TTCGCCACCT CTGACTTGAG CGTCGATTTT TGTGATGCTC GTCAGGGGGG
2451 CGGAGCCTAT GGAAAAACGC CAGCAACGCG GCCTTTTTAC GGTTCCTGGC
2501 CTTTTGCTGG CCTTTTGCTC ACATGTTCTT TCCTGCGTTA TCCCCTGATT
2551 CTGTGGATAA CCGTATTACC GCCTTTGAGT GAGCTGATAC CGCTCGCCGC
2601 AGCCGAACGA CCGAGCGCAG CGAGTCAGTG AGCGAGGAAG CGGAAGAGCG
2651 CCTGATGCGG TATTTTCTCC TTACGCATCT GTGCGGTATT TCACACCGCA
2701 TATGGTGCAC TCTCAGTACA ATCTGCTCTG ATGCCGCATA GTTAAGCCAG
2751 TATACACTCC GCTATCGCTA CGTGACTGGG TCATGGCTGC GCCCCGACAC

```
2801  CCGCCAACAC CCGCTGACGC GCCCTGACGG GCTTGTCTGC TCCCGGCATC
2851  CGCTTACAGA CAAGCTGTGA CCGTCTCCGG GAGCTGCATG TGTCAGAGGT
2901  TTTCACCGTC ATCACCGAAA CGCGCGAGGC AGCAGATCAA TTCGCGCGCG
2951  AAGGCGAAGC GGCATGCATT TACGTTGACA CCATCGAATG GTGCAAAACC
3001  TTTCGCGGTA TGGCATGATA GCGCCCGGAA GAGAGTCAAT TCAGGGTGGT
3051  GAATGTGAAA CCAGTAACGT TATACGATGT CGCAGAGTAT GCCGGTGTCT
3101  CTTATCAGAC CGTTTCCCGC GTGGTGAACC AGGCCAGCCA CGTTTCTGCG
3151  AAAACGCGGG AAAAAGTGGA AGCGGCGATG GCGGAGCTGA ATTACATTCC
3201  CAACCGCGTG GCACAACAAC TGGCGGGCAA ACAGTCGTTG CTGATTGGCG
3251  TTGCCACCTC CAGTCTGGCC CTGCACGCGC CGTCGCAAAT TGTCGCGGCG
3301  ATTAAATCTC GCGCCGATCA ACTGGGTGCC AGCGTGGTGG TGTCGATGGT
3351  AGAACGAAGC GGCGTCGAAG CCTGTAAAGC GGCGGTGCAC AATCTTCTCG
3401  CGCAACGCGT CAGTGGGCTG ATCATTAACT ATCCGCTGGA TGACCAGGAT
3451  GCCATTGCTG TGGAAGCTGC CTGCACTAAT GTTCCGGCGT TATTTCTTGA
3501  TGTCTCTGAC CAGACACCCA TCAACAGTAT TATTTTCTCC CATGAAGACG
3551  GTACGCGACT GGGCGTGGAG CATCTGGTCG CATTGGGTCA CCAGCAAATC
3601  GCGCTGTTAG CGGGCCCATT AAGTTCTGTC TCGGCGCGTC TGCGTCTGGC
3651  TGGCTGGCAT AAATATCTCA CTCGCAATCA AATTCAGCCG ATAGCGGAAC
3701  GGGAAGGCGA CTGGAGTGCC ATGTCCGGTT TTCAACAAAC CATGCAAATG
3751  CTGAATGAGG GCATCGTTCC CACTGCGATG CTGGTTGCCA ACGATCAGAT
3801  GGCGCTGGGC GCAATGCGCG CCATTACCGA GTCCGGGCTG CGCGTTGGTG
3851  CGGATATCTC GGTAGTGGGA TACGACGATA CCGAAGACAG CTCATGTTAT
3901  ATCCCGCCGT CAACCACCAT CAAACAGGAT TTTCGCCTGC TGGGGCAAAC
3951  CAGCGTGGAC CGCTTGCTGC AACTCTCTCA GGGCCAGGCG GTGAAGGGCA
4001  ATCAGCTGTT GCCCGTCTCA CTGGTGAAAA GAAAAACCAC CCTGGCGCCC
4051  AATACGCAAA CCGCCTCTCC CCGCGCGTTG GCCGATTCAT TAATGCAGCT
4101  GGCACGACAG GTTTCCCGAC TGGAAAGCGG GCAGTGAGCG CAACGCAATT
4151  AATGTGAGTT AGCGCGAATT GATCTG
```

Beispiel 1:

Konstruktion der Expressionsvektoren pTrc99A, pTrc99B und pTrc99C

pTrc8g-1 (Euopäische Patentanmeldung EP0236978) wurde mit BglII linarisiert und die überlappenden Enden mittels Klenow-Polymerase aufgefüllt. Die DNA wurde anschließend mit Phosphatase behandelt. Das Plasmid placI$^q$ (Wang et al. (1983) Cold Spring Harbor Symp. Quan. Biol. 47, 85-91) wurde mit EcoRI verdaut, die überlappenden Enden ebenfalls mittels Klenow Polymerase aufgefüllt und das ca. 1100 Basenpaar große Fragment, welches das vollständige lacI$^q$ Allel trägt (Calos (1978) Nature 274, 762-765), wurde mit der linearisierten pTrc89-1 DNA ligiert und transformiert. Das resultierende Plasmid (mit der Transcriptionsrichtung des lacI$^q$ Genes identisch zur Transcriptionsrichtung des trc Promoters) war pTrc90-3. Die Übergänge der Vektor DNA zum lacI$^q$ Fragment wurden sequenziert; diese Information ist in der Sequenz der Tabelle mit enthalten. Die Sequenz von pTrc90-3 umfaßt 4134 Basenpaare. pTrc90-3 wurde mit NcoI und HindIII verdaut und das große Fragment mit den synthetischen Oligonukleotiden A, B oder C in drei getrennten Ansätzen ligiert und anschließend in kompetente Zellen des E.coli K12 Stammes W3110lacI$^q$ transformiert:

```
                    5' CATGGAATTCCGA
Oligo A                   |||||||||
                          CTTAAGGCTTCGA 5'

Oligo B             5' CATGGGAATTCGA
                          |||||||||
                          CCTTAAGCTTCGA 5'

Oligo C             5' CATGGGGAATTCGA
                          ||||||||||
                          CCCTTAAGCTTCGA 5'
```

Die resultierenden Plasmide pTrc98A (Oligo A); pTrc98B (Oligo B) und pTrc98C (Oligo C) wurden mit EcoRI und HindIII verdaut, das große Fragment jeweils isoliert und mit dem 55 Basenpaar großen EcoRI-HindIII Polylinkerfragment des handelsüblichen Vektors pUC18 (Yanisch-Perron et al. (1985) Gene 33, 103-119) ligiert. Die resultierenden Plasmide sind pTrc99A (4176 Basenpaare), pTrc99B (4177 Basenpaare) und pTrc99C (4178 Basenpaare). Als Folge der einligierten synthetischen Oligonukleotide A, B und C unterscheiden sich die Vektoren um jeweils eine Base zwischen der NcoI und der EcoRI Erkennungsstelle, was wiederum in einem Verschieben des Translationsleserasters resultiert. Die Anwesenheit des lacI$^q$ Gens auf dem Expressionsvektor (pTrc99 Serie) ist dann interessant, wenn E.coli Stämme zur Expression benutzt werden sollen, die keinen endogenen lac Repressor besitzen, bzw. in solchen Fällen, in denen das exprimierte Protein toxische Wirkung auf die E.coli Wirtszelle ausübt und aus diesem Grunde eine vollständige Repression in der Klonierungs- bzw. Anzuchtphase erforderlich ist. Das Konstruktionsschema der Vektoren ist in der Abbildung gezeigt.

Beispiel 2:

Expression des Kalzium-bindenden Proteins Calmodulin

Calmodulin ist ein stark konserviertes Kalzium-bindendes Protein, welches ubiquitär in Eukaryonten vorkommt (Baba et al. (1984) Mol. Biol. Evol. 1, 442-455; Klee et al. (1980) Annu. Rev. Biochem. 49, 488-518; Means et al. (1982) Physiol. Rev. 62, 1-39). Es ist involviert in zahlreichen Kalzium-kontrollierten intrazellulären bioregulatorischen Prozessen, einschließlich der Regulation der cyclischen Nukleotide (Cheung (1980) Science 207, 19-27; Klee et al. (1980) Annu. Rev. Biochem. 49, 488-518). Die DNA Sequenz der Calmodulin cDNA, isoliert aus einer Rattenhirn cDNA Genbank, ist bekannt (Sherbany et al (1987) DNA 6, 267-272). Sie kodiert für ein Protein mit 149 Aminosäuren. Obwohl die Calmodulin cDNA Sequenz der Ratte sich in 66 Nukleotiden von der humanen cDNA unterscheidet, zeigen die abgeleiteten Aminosäuresequenzen eine 100%ige Homologie. Dies zeigt, daß es sich um "silent exchanges" handelt und das das Protein in der Tat ein in der Evolution stark konserviertes Protein sein muß. Die Ratten Calmodulin cDNA weist an ihrem Initiationscodon eine NcoI Stelle auf:

```
                     Met Ala Asp Gln
5'... GTGCCTCGCC ATG GCT GAC CAG ...3'
```

Zur Expression der Calmodulin cDNA in E. coli wurde der Klon prCm79 mit NcoI und SmaI verdaut und das 457 Basenpaar große Fragment isoliert und in den entsprechend verdauten Vektor pTrc99A ligiert. Das resultierende Plasmid pTrc99A-Cal exprimiert nach Induktion in E.coli das ca. 17 kD große Calmodulin Protein.

## Ansprüche

1. Expressionsvektoren zur Expression von Fremdproteinen in E.coli enthaltend
   a) den trc-Promotor,
   b) die lacZ Ribosomenbindungsstelle,
   c) eine NcoI-Schnittstelle, die ein Translationsinitiationscodon beinhaltet,
   d) einen Restriktionsenzymschnittstellen-Polylinker in allen drei Leserastern,
   e) sowie Transkriptionsterminatoren,
   f) wobei die vorstehend genannten Elemente in ein Plasmidrückgrat mit Replikationsorigin, Antibiotikaresistenzgen und dem $lacI^q$ Allel integriert sind.

2. Expressionsvektoren nach Anspruch 1 mit pBR322 DNA als Plasmidrückgrat.
3. Expressionsvektoren nach Anspruch 2 mit Deletion der pBR322 DNA bis zur Position 2066.
4. Expressionsvektoren nach Anspruch 1 mit dem EcoRI-HindIII Polylinker aus pUC18.
5. Expressionsvektoren nach Anspruch 1 mit Transkriptionsterminatoren des rrnB Operons.
6. Plasmide pTrc99A, pTrc99B und pTrc99C.
7. Verfahren zur Herstellung von Expressionsvektoren nach Anspruch 1 bis 6, dadurch gekennzeichnet, daß die genannten Elemente (a) bis (f) ligiert werden.
8. Verwendung von Expressionsvektoren nach Anspruch 1 bis 6 zur Expression von Fremdproteinen in E.coli.

Patentansprüche für folgenden Vertragsstaat: ES

1. Verfahren zur Herstellung von Expressionsvektoren zur Expression von Fremdproteinen in E.coli, dadurch gekennzeichnet, daß die Elemente
   a) der trc-Promotor,
   b) die lacZ Ribosomenbindungsstelle,
   c) eine NcoI-Schnittstelle, die ein Translationsinitiationscodon beinhaltet,
   d) ein Restriktionsenzymschnittstellen-Polylinker in allen drei Leserastern,
   e) sowie Transkriptionsterminatoren verbunden werden,
   f) wobei die vorstehend genannten Elemente in ein Plasmidrückgrat mit Replikationsorigin, Antibiotikaresistenzgen und dem $lacI^q$ Allel integriert sind.

2. Verfahren nach Anspruch 1, dadurch gekennzeichnet, daß pBr322 als Plasmidrückgrat eingesetzt wird.
3. Verfahren nach Anspruch 2, dadurch gekennzeichnet, daß die pBR322 DNA bis zur Position 2066 deletiert ist.
4. Verfahren nach Anspruch 1, dadurch gekennzeichnet, daß der EcoRI-HindIII Polylinker aus pUC18 eingesetzt wird.

5. Verfahren nach Anspruch 1, dadurch gekennzeichnet, daß die Transkriptionsterminatoren des rrnB Operons eingesetzt wird.

6. Verfahren zur Expression von Fremdproteinen in E.coli, dadurch gekennzeichnet, daß nach Anspruch 1 bis 5 hergestellte Vektoren eingesetzt werden.

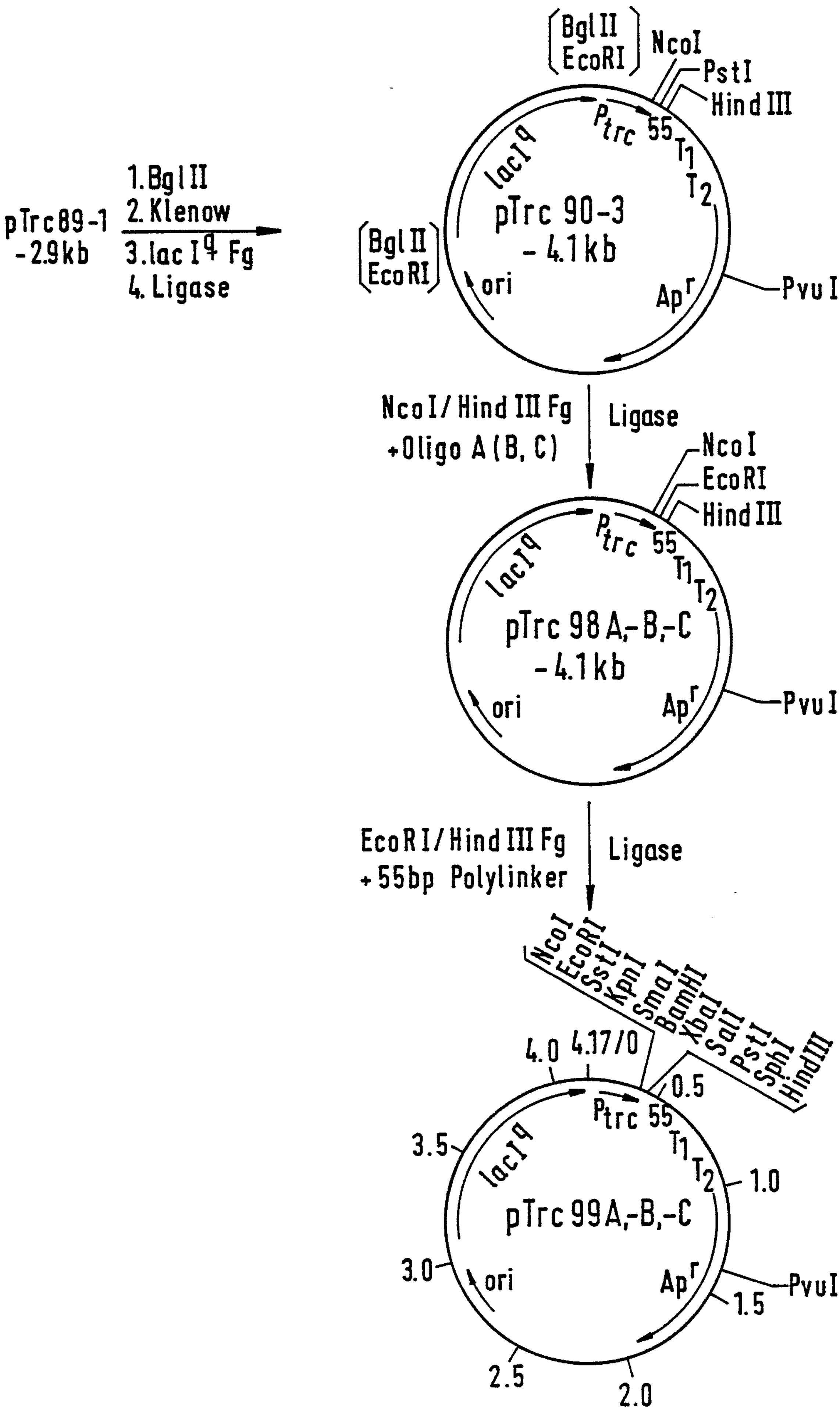
pTrc89-1
-2.9kb
1. Bgl II
2. Klenow
3. lac I^q Fg
4. Ligase
Bgl II
EcoRI
NcoI
PstI
Hind III
Ptrc
55
T1
T2
lacI^q
pTrc 90-3
- 4.1kb
Bgl II
EcoRI
ori
Ap^r
PvuI
NcoI/Hind III Fg
+Oligo A (B, C)
Ligase
NcoI
EcoRI
Hind III
pTrc 98 A,-B,-C
- 4.1kb
EcoRI/Hind III Fg
+55bp Polylinker
Ligase
NcoI
EcoRI
SstI
KpnI
SmaI
BamHI
XbaI
SalI
PstI
SphI
HindIII
4.0
4.17/0
0.5
3.5
1.0
pTrc 99A,-B,-C
3.0
1.5
2.5
2.0